# EUROPEAN PATENT SPECIFICATION

(11) **EP 1 922 302 B1**
(45) Date of publication and mention of the grant of the patent: **23.01.2013**
(21) Application number: 06813558.1
(22) Date of filing: 18.08.2006
(51) Int. Cl.: C07C 303/00, C07C 231/00, C09B 45/14, A01N 59/00, A01N 41/06, A01N 31/02, A01P 1/00, A01P 3/00

(54) **Biocidal use of active aromatic sulfonamide compositions**
Biozide Verwendung von aktiven aromatischen Sulfonamid-Zusammensetzungen
Utilisation en tant que biocides des compositions des sulfamides aromatiques

(30) Priority: 19.08.2005 US 709919 P; 31.08.2005 US 216495; 18.08.2006 US 506737
(43) Date of publication of application: 21.05.2008
(73) Proprietor: Sat, Inc., Union, KY 41091 (US)
(72) Inventor: SCHNEIDER, Charles, A., Villa Hills, KY 41017 (US); SCHNEIDER, David, J., Union, KY 41091 (US)
(74) Representative: Kador & Partner
(86) International application number: PCT/US2006/032469
(87) International publication number: WO 2007/022453

(56) References cited:
- WO-A1-99/65305
- US-A- 2 809 937
- US-A- 5 710 101
- US-A- 5 952 359
- US-A1- 2003 162 755
- US-A1- 2004 265 265
- DATABASE WPI Week 200328 Thomson Scientific, London, GB; AN 2003-281927 XP002595921 & JP 2002 265308 A (ST KAGAKU KK) 18 September 2002 (2002-09-18)
- DATABASE CAPLUS [Online] CHEMICAL ABSTRACTS SERVICE, COLUMBUS, OHIO, US; 1968, CHRZASZEWSKA ANNA ET AL: "Salts of N-halogen amides of benzenesulfonic acid, containing halogen in the phenyl ring, with bi- and trivalent metals" XP008117258 retrieved from CAPLUS Database accession no. 1968:418848

## Description

### BACKGROUND

This disclosure relates to uses of specific halo active aromatic sulfonamide organic compounds which have enhanced biocidal properties and minimal side effects. In a broad context, this disclosure concerns the use of halo active sulfonamide compounds to arrest or kill the growth of living organisms, particularly microorganisms. It may also be used as a fungicide or pesticide.

When the halo active aromatic sulfonamide compounds in this invention are used, solutions of the sulfonamide compound with a low moleculeur weight alcohol are brought into contact with an area affected by microorganisms. This contact is usually affected by spraying, washing, dipping, and/or mixing in such a manner as to contact the affected area, surface, or substrate with an aqueous formulation of the desired sulfonamide compound or a blended mixture of same.

### REFERENCES

U.S. Pat. No. 6,296,841 relates to the use of Chloramine-T, a sulfonamide, as an odor control agent wherein the Chloramine-T is used with a wetting agent. The disclosure relates primarily to domestic odor control.

U.S. Pat. No. 6,743,420 describes the use of Chloramine-T as an odor control agent wherein the Chloramine-T is used with and without a wetting agent. The disclosure of this patent relates to domestic and industrial odor control.

U.S. Pat. No. 6,667,030 further relates to the use of Chloramine T as an odor control agent.
US 2004/0265265A1 discloses halo active sulfonamide organic compounds for controlling unwanted odors.

### BRIEF DESCRIPTION

Disclosed herein, in various embodiments, is the use of biocidal solutions comprising halo active aromatic sulfonamide organic compounds as defined in claim 1 and a low molecular weight alcohol as defined in claim 1 biocides. Processes for using the solutions are also described. The solutions may further comprise a wetting agent

Also disclosed are processes for arresting or killing unwanted microorganisms on an affected area by treating the area with the solution. The solution may be sprayed, swabbed, or vapor contacted. Alternatively, the solution may be incorporated into a soap, lotion, wipe, swab, bath, medical device, dressing, or the like.

These and other non-limiting features or characteristics of the present disclosure will be further described below.

### DETAILED DESCRIPTION

The halo active aromatic sulfonamide compounds as used in this application exhibit enhanced biocidal properties. In addition many of these compounds have very low toxicity properties which make them attractive for use around human, animal and aquatic environments.

The halo active aromatic sulfonamide compounds suitable for use in the solutions of the present application are selected from the Formulas V-VII disclosed further down.

Compounds of Formulas V-VII may or may not be hydrated (n H₂O), but are generally isolated as a trihydrate (where n=3). The compounds of Formulas V-VII are very soluble in water. This property allows for easy compounding of biocidal solutions and allows high percentages of the compounds to be formulated into useful solution products. Furthermore, these compounds have minimal bleach odor. This property is highly advantageous because formulations with strong bleach odor are undesirable in many applications. These compounds have also been found to be stable in the 45-50 °C range for greater than 6 months in water at various concentrations.

The alkali metal salts of N-halo-4-carboxybenzene sulfonamides in particular have been found to be particularly outstanding biocides. These sulfonamides are shown in Formula V:

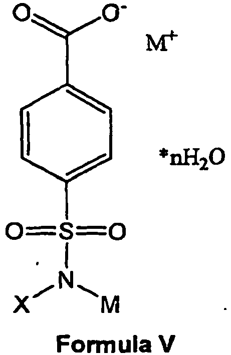

wherein X is a halogen; and

M is an alkali or alkaline earth metal.

In specific embodiments of Formula V, M is sodium or potassium; and X is chlorine. The salts in the dry state are hydrated. The chemical structures of these specific salts are shown in Formulas VI and VII:

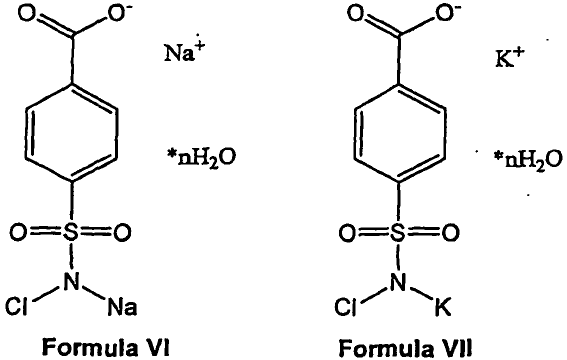

The salts of Formula V have been found to be useful biocides at concentrations of from about 0.01 to about 37% in water. (All concentrations in this application should be interpreted as wt/wt.) Especially useful concentrations are from about 0.1 to about 20%. Water is also preferred as a solvent for the sulfonamide salts of the present application.

These alkali metal salts have been found to function as biocides with minimal undesirable side effects. A particularly beneficial property is the fact that these salts are nontoxic to humans, but toxic to the unwanted organisms. As a result of this nontoxicity, the salts in question can be used in proximity to humans with generally no ill effects. Tests showed that the disodium salts of N-chloro-4-carboxybenzene sulfonamide formulations had zero kill when attempting to establish LD₅₀, and hence they are classified as nontoxic to humans.

In further specific embodiments of the present application, the biocidal solution comprises a wetting agent. The wetting agent aids in placement and functioning of the biocide by ensuring that the sulfonamide salt is able to contact the organism it is being used to kill.

An additional aspect of this application is concerned with the fact that many wetting agents may adversely affect the formation of the Cl+ moiety, from the compounds of Formulas V-VII or degrade said Cl+ moiety once it is formed. Both synthetic and natural wetting agents exist They are generally classified as cationic, anionic, amphoteric and nonionic. Nonionic wetting agents are generally preferred for use in the biocidal solution. However, satisfactory agents may be found in any class of wetting agents.

One especially suitable wetting agent for use in the biocidal solution of the present application is an anionic wetting agent sold under the trademark AVANEL S-74 by the BASF Chemical Co. of Mt. Olive, N.J. AVANEL S-74 appears to be sulfate capped alkyl ethoxylate, where the wetting agent contains 3 units of ethoxylate and the alkyl is a C8 alkyl.

It is unclear how different wetting agents sometimes degrade the Cl+ moiety. It is felt that functional groups such as alkenes, alcohol, ketone, especially aliphatic ketones or aldehydes containing at least one alpha hydrogen next to the carbonyl carbon, and phenols as may be contained on the base wetting agent molecule are particularly harmful to the Cl+ moiety. Impurities as may be contained in various commercially available wetting agents can also play a significant part in the degradation of the Cl+ moiety. Some known degradative impurities are aromatic and conjugated phenols, compounds containing activated carbonyl, alpha aliphatic hydrogens, or active primary and secondary amines.

The concentration of the wetting agent in the biocidal solutions of the present application can be from about 0.05 to about 5%. In more specific embodiments, the concentration for the wetting agent is from about 0.5 to about 1.5%.

Another factor in choosing the concentration of the wetting agent is the degree to which it foams. If undesirable foaming occurs, anti foamers may be added to the solution.

The biocidal solution as used according to the present invention further comprises a low molecular weight alcohol selected from the group consisting of methanol, t-butanol, ethanol and isopropanol. The combination of sulfonamide and alcohol appears to synergistically enhance the biocidal effect of the solution. However, suitable alcohols are limited. Alcohols which do not contain hydrogen atoms alpha to the -OH moiety appear to offer more stable formulations. Alpha hydrogen atoms appear to reduce stability due to interaction with the active halogen contained in the active aromatic halo sulfonamide. Specific alcohols which are suitable for use include methanol and t-butanol [C(CH₃)₃OH]. In addition, t-butanol has a pleasant odor which may be pleasing to the consumer.

The alcohol is present in the biocidal solution at concentrations of from about 0.1 to about 5.0%. In more specific embodiments, the alcohol is present at a concentration of from about 0.2 to about 1.0%.

The pH of the biocidal solution should be maintained within a range of from about 6.5 to about 10.0. In specific embodiments, the solution has a pH of from about 6.5 to about 9.5. In more specific embodiments, the solution has a pH of from about 7.0 to about 9.0. In still more specific embodiments, the solution has a pH of from about 7.5 to about 8.5. One consideration is the fact that aromatic N-halo active sulfonamide compounds exhibit excellent stability in a pH range of 8-9.5, which is important when long shelf life is very desirable.

Buffering agents may be used to maintain the pH within the desired range. They may also allow the active ingredients of the biocidal solution to be shipped in powdered form and mixed by the consumer with no adverse effect Suitable buffering agents include sodium bicarbonate, potassium bicarbonate, sodium carbonate, potassium carbonate, acetate buffers (such as sodium acetate), phosphate buffers, pH blended phosphates, and sulfate buffers.

The concentration of the buffering agent can be from 0.1% up to the limit of solubility. The preferred range for the concentration of the buffering agent is from about 5% to about 200% of the active compound in solution. A more preferred range is from about 5% to about 50% with a most preferred concentration being 25-50%.

Unwanted troublesome microorganisms may be killed by various uses of the biocidal solution. A solution of the defined biocide may be sprayed onto an infected substrate. It can be wiped or swabbed onto an affected area. The solution can also be incorporated into a soap, lotion or the like. It could also be incorporated into a bath. Moreover, it may be incorporated into a medical device or dressing or related useful items.

The use of the biocidal solution of the present application is illustrated by the following non-limiting examples, it being understood that these examples are intended to be illustrative only and that the present application is not intended to be limited to the materials, conditions, process parameters and the like recited herein. All proportions are by weight unless otherwise indicated.

### EXAMPLES

In order to demonstrate the biocidal effects of the biocidal solution of the present application, a 1 % aqueous solution of the sodium salt of Formula VI described above was sprayed on bacteria-laden test panels. The solution further contained 0.2% sodium bicarbonate and 0.03% of a wetting agent sold under the trademark AVANEL-S74. The exposure time and % kill of the test are set forth in Table I.

**Table I. Organism (as %killed)**

| Exposure Time | Staph Epi | E. coli | VRE | MRSA | Pseudomonas | Strep A |
|---|---|---|---|---|---|---|
| 10 min | 100 | 100 | 100 | 100 | 100 | 100 |
| 5 min | 100 | 100 | 100 | 100 | 100 | 100 |
| 1 min | 99.6 | 100 | 91.4 | 99.2 | 100 | 72.1 |
| 30 sec | 88.7 | 100 | 81.4 | 91.2 | 100 | 68.9 |
| 5 sec | 8.1 | 86.9 | 40.9 | 66.7 | 55.1 | 59.5 |

Toxicity studies of N-chloro-4-carboxybenzene sulfonamide were also performed. In single dose oral toxicity studies with 10 rats using an aqueous solution of the compound, toxicity was determined to be greater than 5,000 mg/kg. In a skin irritation study conducted on rabbits using a formulation containing the compound, there was no incidence of irritation over 1, 24, 48, or 72 hour periods.

The aqueous solution comprising 1% of the sodium salt of Formula VI, 0.2% sodium bicarbonate and 0.03% AVANEL-S74 was then mixed with various alcohols to determine which alcohols best enhanced the biocidal effect of the solution. The alcohol was added at 1% concentration. The samples were held at room temperature for 3 days, then heated and held at 50°C and checker - periodically for active chlorine. The results are set forth in Table II as the % of active chlorine.

**Table II. % Active Chlorine**

| Time -> | Initial | 7 days | 14 days | 21 days | 39 days | 63 days | % active chlorine remaining |
|---|---|---|---|---|---|---|---|
| Methanol | 0.21 | 0.191 | 0.17 | 0.16 | 0.12 | 0.09 | 43% |
| Isopropyl alcohol | 0.21 | 0.12 | 0.03 | 0.004 | 0 | 0 | 0 |
| Ethanol | 0.21 | 0.2 | 0.18 | 0.14 | 0.004 | 0 | 0 |
| t-butanol | 0.21 | 0.2 | 0.2 | 0.2 | 0.19 | 0.19 | 90% |
| 1-propanol | 0.21 | 0.1 | 0 | 0 | 0 | 0 | 0 |
| 1-butanol | 0.21 | 0.1 | 0 | 0 | 0 | 0 | 0 |
| phenol | 0.21 | 0 | 0 | 0 | 0 | 0 | 0 |

## Claims

1. Use of an effective amount of a halo active aromatic sulfonamide compound of the following Formula V: 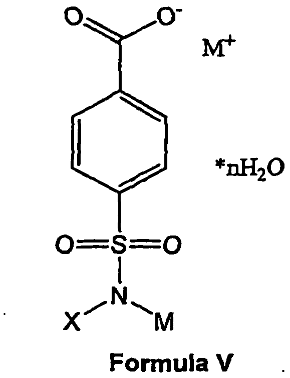 wherein X is a halogen; and
M is an alkali or alkaline earth metal
for arresting unwanted microorganisms on an affected area, comprising:
contacting the area with a biocidal solution comprising said compound and a low molecular weight alcohol selected from the group consisting of methanol, t-butanol, ethanol and isopropanol in a concentration from 0.1 to 5.0% by weight.

2. Use of claim 1, wherein the solution further comprises a wetting agent that essentially does not react with the sulfonamide compound.

3. Use of claim 2, where the wetting agent is a nonionic wetting agent.

4. Use of claim 2, where the wetting agent is a sulfate capped alkyl ethoxylate.

5. Use of claim 2, wherein the concentration of the wetting agent in the biocidai solution is from about 0.05 to about 5%.

6. Use of claim 1, wherein the solution is buffered to a pH of from about 6.5 to about 10.0.

7. Use of claim 6, wherein the solution is buffered to a pH of from about 7.0 to about 9.0.

8. Use of claim 7, wherein the solution is buffered to a pH of from about 7.5 to about 8.5.

9. Use of claim 6, wherein the solution is buffered with sodium bicarbonate.

10. Use of claim 1, wherein the concentration of the sulfonamide compound in the biocidal solution is from about 0.1 to about 37%.

11. Use of claim 10, wherein the concentration of the sulfonamide compound in the biocidal solution is from about 0.1 to about 20%.

12. Use of claim 1, wherein the biocidal solution is incorporated into a soap, lotion, wipe, or swab which is then contacted with the affected area.

13. Use of claim 1, wherein the sulfonamide compound is of the following Formula VI:

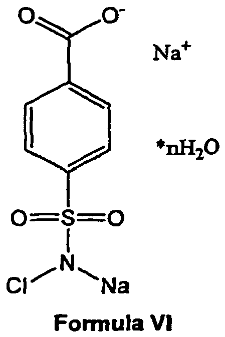

14. Use of claim 1, wherein the sulfonamide compound is of the following Formula VII:

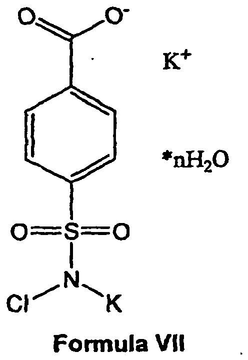

## Patentansprüche

1. Verwendung einer wirksamen Menge einer halogenaktiven aromatischen Sulfonamidverbindung der folgenden Formel V: 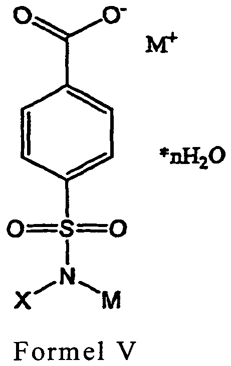 worin X ein Halogen ist und
M ein Alkali- oder Erdalkalimetall ist,
für die Hemmung unerwünschter Mikroorganismen auf einem befallenen Bereich, die folgendes aufweist:
Inkontaktbringen des Bereichs mit einer bioziden Lösung, die diese Verbindung und einen Alkohol mit geringem Molekulargewicht, der aus der Gruppe ausgewählt ist, die aus Methanol, t-Butanol, Ethanol und Isopropanol besteht, in einer Konzentration von 0,1 bis 5,0 Gew.-% aufweist.

2. Verwendung nach Anspruch 1, wobei die Lösung ferner ein Benetzungsmittel aufweist, das im wesentlichen nicht mit der Sulfonamidverbindung reagiert.

3. Verwendung nach Anspruch 2, wobei das Benetzungsmittel ein nichtionisches Benetzungsmittel ist.

4. Verwendung nach Anspruch 2, wobei das Benetzungsmittel ein mit Sulfat geschütztes Alkylethoxylat ist.

5. Verwendung nach Anspruch 2, wobei die Konzentration des Benetzungsmittels in der bioziden Lösung etwa 0,05 bis etwa 5 % beträgt.

6. Verwendung nach Anspruch 1, wobei die Lösung auf einen pH-Wert von etwa 6,5 bis etwa 10,0 gepuffert ist.

7. Verwendung nach Anspruch 6, wobei die Lösung auf einen pH-Wert von etwa 7,0 bis etwa 9,0 gepuffert ist.

8. Verwendung nach Anspruch 7, wobei die Lösung auf einen pH-Wert von etwa 7,5 bis etwa 8,5 gepuffert ist.

9. Verwendung nach Anspruch 6, wobei die Lösung mit Natriumbicarbonat gepuffert ist.

10. Verwendung nach Anspruch 1, wobei die Konzentration der Sulfonamidverbindung in der bioziden Lösung etwa 0,1 bis etwa 37 % beträgt.

11. Verwendung nach Anspruch 10, wobei die Konzentration der Sulfonamidverbindung in der bioziden Lösung etwa 0,1 bis etwa 20 % beträgt.

12. Verwendung nach Anspruch 1, wobei die biozide Lösung in einer Seife, Lotion, ein Abwischmittel oder einen Tupfer aufgenommen wird, die dann mit dem befallenen Bereich in Kontakt gebracht werden.

13. Verwendung nach Anspruch 1, wobei die Sulfonamidverbindung die folgende Formel VI hat:

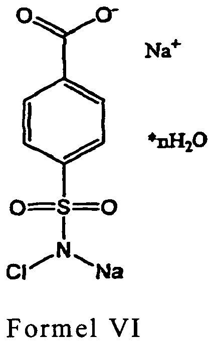

14. Verwendung nach Anspruch 1, wobei die Sulfonamidverbindung die folgende Formel VII hat:

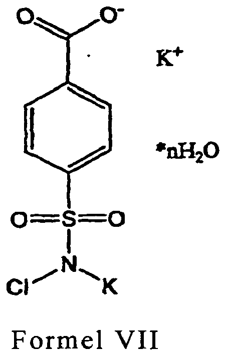

## Revendications

1. Utilisation d'une quantité efficace d'un composé sulfamide aromatique actif halogéné répondant à la Formule V suivante : 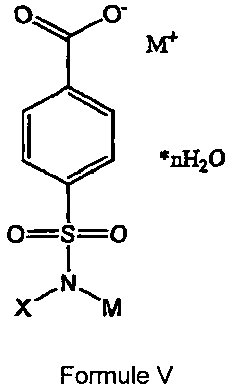 dans laquelle X est un halogène ; et
M est un métal alcalin ou alcalino-terreux
pour lutter contre des microorganismes indésirables sur une zone affectée, comprenant :
la mise en contact de la zone avec une solution biocide comprenant ledit composé et un alcool de faible poids moléculaire choisi dans le groupe constitué du méthanol, du t-butanol, de l'éthanol et de l'isopropanol dans une concentration de 0,1 à 5,0 % en poids.

2. Utilisation selon la revendication 1, dans laquelle la solution comprend en outre un agent mouillant qui ne réagit quasiment pas avec le composé sulfamide.

3. Utilisation selon la revendication 2, dans laquelle l'agent mouillant est un agent mouillant non ionique.

4. Utilisation selon la revendication 2, dans laquelle l'agent mouillant est un éthoxylate d'alkyle couvert de sulfate.

5. Utilisation selon la revendication 2, dans laquelle la concentration de l'agent mouillant dans la solution biocide est d'environ 0,05 à environ 5 %.

6. Utilisation selon la revendication 1, dans laquelle la solution est tamponnée à un pH d'environ 6,5 à environ 10,0.

7. Utilisation selon la revendication 6, dans laquelle la solution est tamponnée à un pH d'environ 7,0 à environ 9,0.

8. Utilisation selon la revendication 7, dans laquelle la solution est tamponnée à un pH d'environ 7,5 à environ 8,5.

9. Utilisation selon la revendication 6, dans laquelle la solution est tamponnée avec du bicarbonate de sodium.

10. Utilisation selon la revendication 1, dans laquelle la concentration du composé sulfamide dans la solution biocide est d'environ 0,1 à environ 37 %.

11. Utilisation selon la revendication 10, dans laquelle la concentration du composé sulfamide dans la solution biocide est d'environ 0,1 à environ 20 %.

12. Utilisation selon la revendication 1, dans laquelle la solution biocide est incorporée dans un savon, une lotion, une lingette ou un tampon qui est alors mis en contact avec la zone affectée.

13. Utilisation selon la revendication 1, dans laquelle le composé sulfamide répond à la Formule VI suivante :

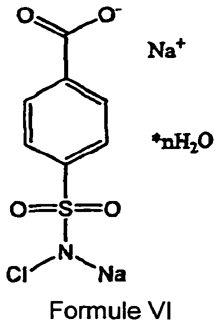

14. Utilisation selon la revendication 1, dans laquelle le composé sulfamide répond à la Formule VII suivante :
